# EUROPEAN PATENT SPECIFICATION

(11) **EP 2 182 948 B1**
(45) Date of publication and mention of the grant of the patent: **06.03.2013**
(21) Application number: 08786340.3
(22) Date of filing: 23.07.2008
(51) Int. Cl.: A61K 31/444, A61K 31/4709, A61K 45/06, A61P 35/00

(54) **USE OF IMIDAZOQUINOLINES FOR THE TREATMENT OF EGFR DEPENDENT DISEASES OR DISEASES THAT HAVE ACQUIRED RESISTANCE TO AGENTS THAT TARGET EGFR FAMILY MEMBERS**
VERWENDUNG VON IMIDAZOCHINOLINEN ZUR BEHANDLUNG VON EGFR-ABHÄNGIGEN KRANKHEITEN ODER KRANKHEITEN MIT ERWORBENER RESISTENZ GEGENÜBER WIRKSTOFFEN, DIE AUF EGFR-FAMILIENMITGLIEDER GERICHTET SIND
UTILISATION D'IMIDAZOQUINOLINES POUR LE TRAITEMENT DE MALADIES DÉPENDANT DE EGFR OU DE MALADIES QUI ONT ACQUIS UNE RÉSISTANCE À DES AGENTS QUI CIBLENT DES MEMBRES DE LA FAMILLE EGFR

(30) Priority: 24.07.2007 EP 07112998
(43) Date of publication of application: 12.05.2010
(73) Proprietor: Novartis AG, 4056 Basel (CH)
(72) Inventor: GARCIA-ECHEVERRIA, Carlos, CH-4052 Basel (CH); MAIRA, Sauveur-Michel, F-68440 Habsheim (FR)
(74) Representative: Gruber, Markus
(86) International application number: PCT/EP2008/059642
(87) International publication number: WO 2009/013305

(56) References cited:
- WO-A-2005/054238
- WO-A-2006/122806
- ZHAO JEAN J ET AL: "The p110alpha isoform of PI3K is essential for proper growth factor signaling and oncogenic transformation." PROCEEDINGS OF THE NATIONAL ACADEMY OF SCIENCES OF THE UNITED STATES OF AMERICA 31 OCT 2006, vol. 103, no. 44, 31 October 2006 (2006-10-31), pages 16296-16300, XP002462087 ISSN: 0027-8424 cited in the application
- IVANOV VLADIMIR N ET AL: "Combined treatment with EGFR inhibitors and arsenite upregulated apoptosis in human EGFR-positive melanomas: a role of suppression of the PI3K-AKT pathway." ONCOGENE, vol. 24, no. 4, 20 January 2005 (2005-01-20), pages 616-626, XP002462088 ISSN: 0950-9232
- BIANCO ET AL: "Rational bases for the development of EGFR inhibitors for cancer treatment" INTERNATIONAL JOURNAL OF BIOCHEMISTRY AND CELL BIOLOGY, EXETER, GB, vol. 39, no. 7-8, 10 July 2007 (2007-07-10), pages 1416-1431, XP022145781 ISSN: 1357-2725

## Description

The present disclosure describes the use of the compound 2-methyl-2-[4-(3-methyl-2-oxo-8-quinolin-3-yl-2,3-dihydro-imidazo[4,5-c]quinolin-1-yl)-phenyl]-propionitrile (Compound A) or a tautomer thereof, or a pharmaceutically acceptable salt, or a hydrate or solvate thereof, in the treatment of Epidermal Growth Factor Receptor (EGFR) family members (including EGFR1 also known as HER1 or Erb-B1; EGFR2 also known as HER2 or Erb-B2; and EGFR3 also known as HER3 or Erb-B3) dependent diseases or diseases that have acquired resistance to agents that target EGFR family members, use of said compound, or a tautomer thereof, or a pharmaceutically acceptable salt, or a hydrate or solvate thereof, for the manufacture of pharmaceutical compositions for the treatment of said diseases, combinations of said compounds with EGFR modulators for said use, methods of treating said diseases with said compounds, and pharmaceutical preparations for the treatment of said diseases comprising said compounds, alone or in combination, especially with an EGFR modulator.

Somatic mutations in the tyrosine kinase domain of EGFR has been associated with the clinical response to EGFR tyrosine kinase inhibitor such as Gefitinib (fressa®) or Erlotinib (Tarceva®) (Paez at al., EGFR mutations in lung cancer: correlation with clinical response to gefitinib therapy, science, vol 304, 1497-1500). Acquired resistance to EGFR modulators occurs in patients who initially responded clinically to therapy, but then developed progressive tumors. Refractory response to EGFR kinase inhibitors is exemplified with the secondary resistant mutation T790M (Kobayashi of al; EGFR mutation and resistance of non-small cell lung cancer to gefitinib, N. Engl J Med, Vol 352, 786-792); which is comparable to the resistance mutation(s) observed for Gleevec/Glivec or Dasatininb in chronic myelogenous leukemia (CML) *(*Gorre et al.; Bcr-Abl point mutants isolated from patients with imatinib mesylate resistant chronic leukemia reamin sensitive to inhibitors of the Bcr-Abl chaperone heat shock protein 90, Blood, vol 100, 3041-3044*)* or GIST patients. *(*Antonescu et al.; Acquired resistance to Imatinib in gastrointestinal stromal tumors occurs through secondary gene mutation, Clin Cancer Res, Vol 11, 4182-4190*).*

Evidences that activation of the PI3K pathway downstream of activated EGFR exists in the literature. Thus, genetic ablation of the PI3K catalytic subunit (p110) in mouse embryo fibroblast renders cells resistant for transformation by an activated form of EGFR (Zhao et al.; The p110 alpha isoform of PI3K is essential for proper growth factor signaling and oncogenic transformation, PEAS, vol 103, 16296-16300*).* HER3 (ErbB-3), one of the four member of the EGFR family and partner of HER1 (EGFR1) is often overexpressed in EGFR inhibitors sensitive tumors, and that is correlated with constitutive PI3K recruitment and activation (Engelman et al.; ErbB-3 mediates phosphoinositide 3-kinase activity In gefitinib-sensitive non small cell lung cancer cell lines, PNAS vol 102, 3788-3793*; Sergina et al.; Escape from HER-family tyrosine kinase inhibitor therapy by the kinase-inactive HER 3*). The genetic and biochemical characterization of tumor biopsies and tumor cell lines harboring EGFR amplification and EGFR inhibitor resistance have revealed a constitutive activation status of the PI3K pathway (Engelman et al.; Allelic disruption obscures detection of a biologically significant resistance mutation in EGFR amplified lung cancer, The Journal of Clinical investigation, vol 116, 2695-2706).

Zhao Jean J et al., Proceedings of the National Academy of Sciences of the United States of America, 31 October 2006, vol. 103, no. 44, pages 16296-16300, describe experiments involving mice carrying a conditionally targeted allele of the PI3KCA gene and suggest that the p110α isoform of PI3K is essential for proper growth factor signaling and oncogenic transformation.

Ivanov Vladimir N et al., Oncogene, 20 January 2005, vol. 24, no. 4, pages 616-626), disclose the effects of a combined treatement of several EGFR inhibitors and arsenite on human EGFR-positive melanomas.

Bianco et al. (International Journal of Biochemistry and Cell Biology, 10 July 2007, vol. 39, no. 7-8, pages 1416-1431) review the role of EGFR in carcinogenesis and tumor progression and discuss a number of monoclonal antibodies and some small molecules acting as tyrosine kinase inhibitors.

WO2005/054238 discloses certain imidazoquinolines and salts thereof which are described as being useful in the treatment of protein kinase dependent diseases.

Surprisingly, it has been found that specific imidazoquinoline derivatives, which have been described in WO2006/122806 provoke strong anti-proliferative activity and an in vivo antitumor response of breast and lung cancer cell lines with amplified EGFRs and/or mutated EGFR1 as single agent and in combination with EGFR kinase modulators. Therefore, said compounds are usefull for the treatment of EGFR dependent disease.

Specific imidazoquinoline derivatives their preparation and suitable pharmaceutical formulations containing the same are described in WO2006/122806 and include compounds of formula I 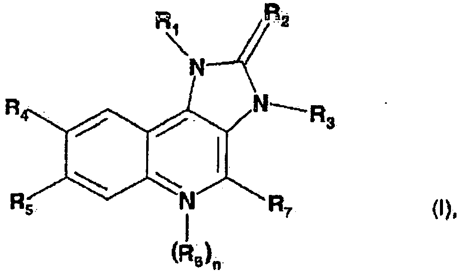 wherein
R₁ is naphthyl or phenyl wherein said phenyl is substituted by one or two substituents independently selected from the group consisting of Halogen; lower alkyl unsubstituted or substituted by halogen, cyano, imidazolyl or triazolyl; cycloalkyl; amino substituted by one or two substituents independently selected from the group consisting of lower alkyl, lower alkyl sulfonyl, lower alkoxy and lower alkoxy lower alkylamino; piperazinyl unsubstituted or substituted by one or two substituents independently selected from the group consisting of lower alkyl and lower alkyl sulfonyl; 2-oxo-pyrrolidinyl; lower alkoxy lower alkyl; imidazolyl; pyrazolyl; and triazolyl;
R₂ is O or S;
R₃ is lower alkyl;
R₄ is pyridyl unsubstituted or substituted-by halogen, cyano, lower alkyl, lower alkoxy or piperazinyl unsubstituted or substituted by lower alkyl; pyrimidinyl unsubstituted or substituted by lower alkoxy; quinolinyl unsubstituted or substituted by halogen; quinoxalinyl; or phenyl substituted with alkoxy
R₅ is hydrogen or halogen;
n is 0 or 1 ;
R₈ is oxido;
with the proviso that if n=1, the N-atom bearing the radical R₈ has a positive charge;
R₇ is hydrogen or amino;
or a tautomer thereof, or a pharmaceutically acceptable salt, or a hydrate or solvate thereof.

The radicals and symbols as used in the definition of a compound of formula I have the meanings as disclosed in WO2006/122806.

The compound of the present invention is a compound which is specifically described in WO2006/122806, ie, the compound 2-methyl-2-[4-(3-methyl-2-oxo-8-quinolinn-3-yl-2,3-dihydro-imidazo[4,5-c]quinolin-1-yl)-phenyl(propionitrile (Compound A) or a tautomer thereof, or a pharmaceutically acceptable salt, or a hydrate or solvate thereof.

A very preferred compound of the present invention is 2-methyl-2-[4-(3-methyl-2-oxo-8-quinolin-3-yl-2,3-dihydro-imidazo[4,5-c]quinolin-1-yl)-phenyl]-propionitrile (Compound A) and its monotosylate salt. The synthesis of 2-methyl-2-[4-(3-methyl-2-oxo-8-quinolin-3-yl-2,3-dihydro-imidazo[4,5-c]quinolin-1-yl)-phenyl]-propionitrile is for instance described in WO2006/12280 as Example 1.

Compounds that target members of the EGFR family according to the present disclosure include EGFR family kinase modulators, compounds that alter EGFR expression levels or elicit a cellular immune reponse linked to the expression of EGFR family members in the tumor cells. Preferrable EGFR modulators exhibit their activity as inhibitors of EGFR functional activity. Compounds that target members of the EGFR family according to the present disclosure include without limitation gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922.

### Short description of the figures

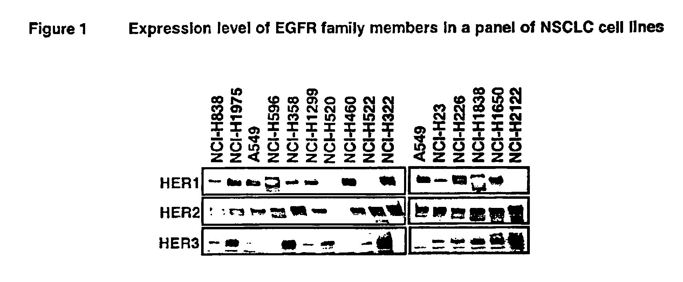

Figure 1 shows the level of expression of EGFR family proteins HER-1, -2 and -3, in a panel of 15 NSCLC human tumor cell lines.
   The indicated tumor lines are cultured in optimal growth conditions, and cell extracts prepared at sub-confluent stage. Equivalent total protein extracts are then subjected to SDS-PAGE, gels transferred and membranes incubated with antibodies raised against the proteins indicated on the left side of the panels.
Figure 2 shows the antitumor activity of Compound A in combination with NVP-AEE788 against NCI-H358 tumors.
   Female Harlan athymic mice (n = 8), bearing s.c NCI-H358 tumors are treated p.o. either with the PI3K inhibitor Compound A or with the EGFR inhibitor NVP-AEE788, or in combination, at the indicated dose and schedule, * p<0.05 (Dunnet's vs controls).
Figure 3 shows the antitumor activity of Compound A against the EGFR inhibitor resistant NSCLC cell line NCl-H1975
   Female Harlan athymic mice (n = 8), bearing s.c NCl-H1975 tumors are treated p.o. with the PI3K inhibitor Compound A or with the EGFR inhibitors NVP-AEE788 and erlotinib at the indicated dose and schedule. *p<0.05 (Dunnet's vs controls).
Figure 4 shows the level of expression of EGFR family proteins HER-1, -2 and -3, in a panel of 15 breast cancer human tumor cell lines
   The indicated tumor lines are cultured in optimal growth conditions, and cell extracts prepared at sub-confluent stage. Equivalent total protein extracts are then subjected to SDS-PAGE, gels transferred and membranes incubated with antibodies raised against the proteins indicated on the left side of the panels.

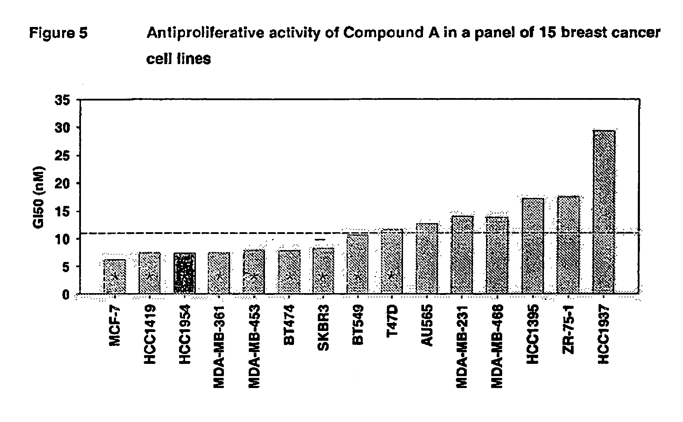

Figure 5 shows the antiproliferative activity of Compound A in a panel of breast cancer cell lines.
   The indicated cell lines are incubated with increasing amount of Compound A, and effect on the proliferation assessed with a Methylene blue viable cell detection assay. The cell lines indicated with a red asterisk are the ones in which cell death is observed (i.e. for which the absorbance observed for treated cells was lower than the original inoculum).

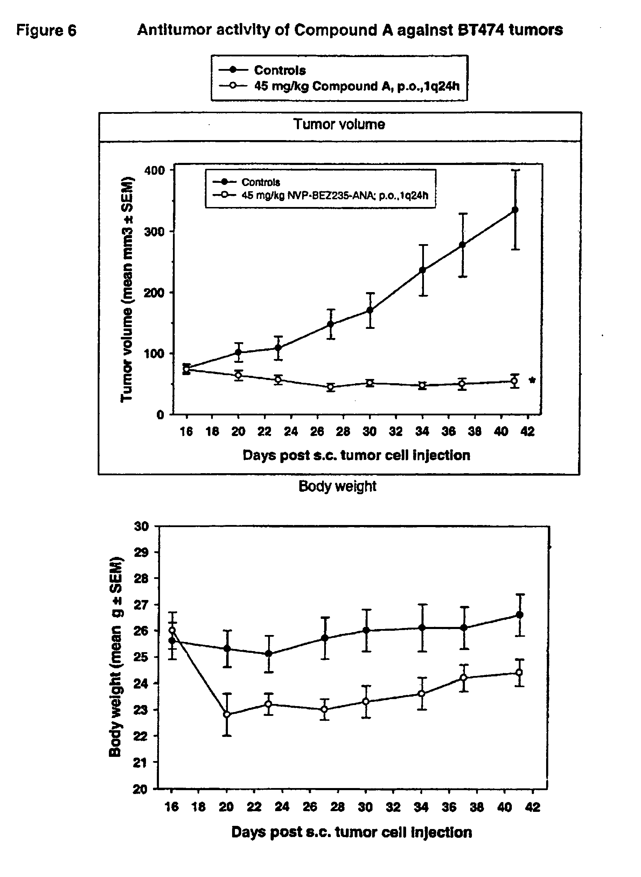

Figure 6 shows the antitumor activity of Compound A against BT474 tumors.
   Female Harlan athymic mice (n = 8), bearing s.c. BT474 tumors are treated p.o. with the PI3K inhibitor Compound A at the indicated dose and schedule.* p<0.05 (Dunnet's).

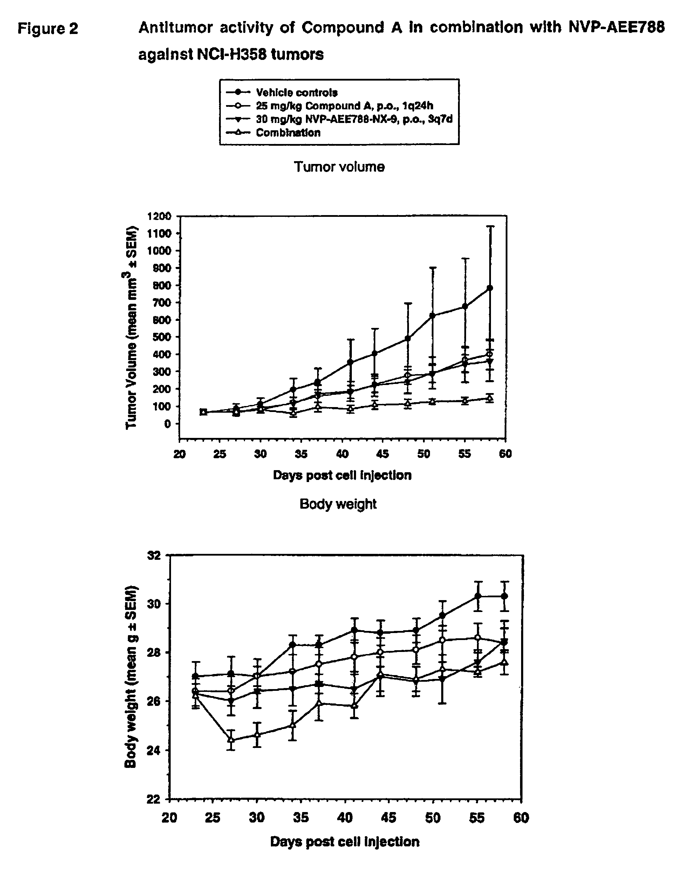

A panel of 15 NSCLC tumors cell lines have been characterized for the expression of the EGFR family members (Figure 1). Consistent with the data described in the literature, most of them shows high levels of HER1 and 3. This is the case of the NCl-H358 cell line that has also been described as sensitive to EGFR kinase inhibitors. This cell line has been tested with EGFR low-molecular mass kinase inhibitors and compounds of formula I. The Growth Inhibitory 50 (GI₅₀) found for gefitinib In a proliferation assay - methylene blue assay - with this cell line, is 542 nM and the GI₅₀ for Compound A is 31 nM. The Combination Index for Compound A and gefitinib in a proliferation assay with NCl-H358 tumor cells is 1.0, reflecting the additive effect of both molecules in this lung tumor cell line. Similar results are obtained with other EGFR inhibitors including NVP-AEE788, erlotinib and lapatinib. Moreover, in vivo combination of such imidazoquinoline derivatives with the EGFR kinase inhibitor NVP-AEE788 results in tumor statis when such compounds are administered to animals bearing subcutaneous NCl-H358 xenografts (Figure 2).

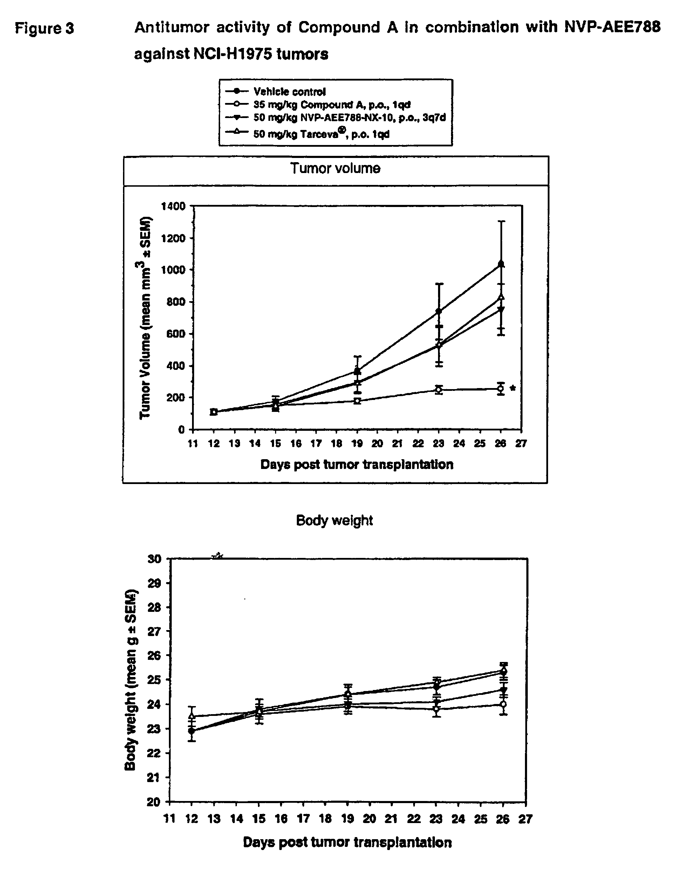

Non small cell lung carcinoma cell lines with amplified EGFR, but refractory to EGFR inhibition therapy, are valuable models to test the sensitivity of PI3K inhibitors like the compounds of formula I in such genetic background. The NCl-H1975 cell line is such a model, as it overexpresses HER1 and HER3 and is highly tumorgenic in vivo. Moreover, it contains HER1 bearing the T790M gatekeeper mutation that renders the kinase resistant to catalytic inhibition. The GI₅₀s in this cell line-are 11.4 nM and 3645 nM for Compound A and gefitinib, respectively. The *in vivo* antitumor activity of PI3K inhibitors like the compounds of formula I is tested against this EGFR driven and inhibitor resistant tumor model (Figure 3). As expected, the EGFR kinase inhibitors NVP-AEE788 and erlotinib show no significant inhibition on tumor growth, but surprisingly administration of Compound A causes *in vivo* tumor growth inhibition. Compound A is well tolerated - no statistically significant difference in body weight between the control and treatment groups can be observed.

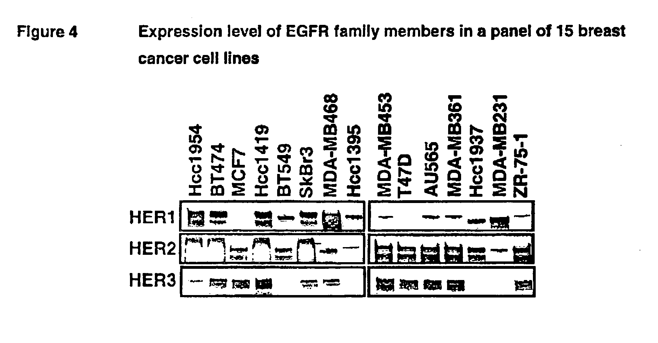

ErB-B2 (HER2) is often overexpressed in breast and ovarian cell lines. The level of expression of this protein in a panel of 15 breast cancer cell lines is shown in Figure 4. Although effective therapeutic approaches exist against ErbB2, 50% of patients with amplified/overexpressed HER2 do not respond to ErbB2 modulators such as trastuzumab. In a panel of breast tumor cell lines containing or not ErbB2 amplification, Compound A decreases cell proliferation with an median GI₅₀ of 11.1 nM, and induces cell death in cell lines that overexpressed ErbB2 (Figure 5). Moreover, BT474 subcutaneous xenografts are exquisitely sensitive to Compound A treatment, as tumor regression is observed upon daily treatment with the compound at a 45 mg/kg, given po, once per day (Figure 6).

A compound of formula I, especially Compound A, is therefore useful for the treatment of such EGFR dependent diseases, especially malignancies, or EGFR family members acquired resistance driven diseases. Diseases or malignancies with an established or potential molecular link to dysregulation of EGFR activity are, for instance, described in *"*Mendelsohn and Baselga; Status of Epidermal Growth Factor Receptor Antagonists in the Biology and Treatment of Cancer, Journal of Clinical Oncology, 2787-2799*'; "*Mendelsohn and Baselga; Epidermal Growth Factor Receptor Targeting in Cancer, Seminars in Oncology, Vol 33, 369-385*";* Irmer et al., EGFR kinase domain mutations - functional impact and relevance for lung cancer therapy, Oncogene, 1-9*;* Roche-Lima et al., EGFR targeting of solid tumors; Cancer Control, 2007, Vol 14 (3), 295-304).

According to the present invention the treatment of the following EGFR dependent diseases, with the compound 2-methyl-2-[4-(3-methyl-2-oxo-8-quinolin-3-yl-2,3-dihydro-imidazo[4,5-c]guinolin-1-yl)-phenyl]-propionitrile (Compound A) or a tautomer thereof, or a pharmaceutically acceptable salt, or a hydrate or solvate thereof provided :
- non small cell lung carcinoma
- head and neck cancer
- colorectal carcinoma
- breast cancer
- brain malignancies including glioblastoma
- prostate cancer
- bladder cancer
- renal cell carcinoma
- pancreas cancer
- cervical cancer
- esophageal cancer
- gastric cancer
- ovarian cancer
or any combination thereof.

The present invention relates to the use of the compound 2-methyl-2-[4-(3-methyl-2-oxo-8-quinolin-3-yl-2,3-dihydro-imidazo[4,5-c]quinolin-1-yl)-phenyl]-propionitrile (Compound A) or a tautomer thereof, or a pharmaceutically acceptable salt, or a hydrate or solvate thereof, for the manufacture of a pharmaceutical preparation for the treatment of an EGFR dependent disease which is:
- non small cell lung carcinoma
- head and neck cancer
- colorectal carcinoma
- breast cancer
- brain malignancies including glioblastoma
- prostate cancer
- bladder cancer
- renal cell carcinoma
- pancreas cancer
- cervical cancer
- esophageal cancer
- gastric cancer
- ovarian cancer
or any combination thereof.

Furthermore, the present disclosure describes the use of a compound of the compound 2-methyl-2-[4-(3-methyl-2-oxo-8-quinolin-8-quinolin-3-yl-2,3-dihydro-imidazo[4,5-c]quinolin-1-yl)-phenyl]-propionitrile (Compound A) or a tautomer thereof, or a pharmaceutically acceptable salt, or a hydrate or solvate thereof, for the manufacture of a pharmaceutical preparation for the treatment of a EGFR dependent disease or malignancy or a disease that has acquired resistance to agents that target EGFR family members.

The resistance to the treatment with an EGFR modulator can been acquired during treatment with said EGFR modulator or can be due to a mutation or mutations in the protein.

Thus, there is provided the compound 2-methyl-2-[4-(3-methyl-2-oxo-8-quinolin-3-yl-2,3-dihydro-imidazo14,5-c]quinolin-1-yl)-phenyl]-propionitrile (Compound A) or a tautomer thereof, or a pharmaceutically acceptable salt, or a hydrate or solvate thereof.
for use in the treatment of an EGFR dependent disease or disease that has acquired resistance during treatment with an EGFR modulator, wherein the disease to be treated is non small cell lung carcinoma, head and neck cancer, colorectal carcinoma, breast cancer, brain malignancies including glioblastoma, prostate cancer, bladder cancer, renal cell carcinoma, pancreas cancer cervical cancer, esophageal cancer, gastric cancer ovarian cancer or any combination thereof.

In particular, the present disclosure describes the treatment of a disease or malignancy that is dependent on EGFR family members or has aquired resistance during treatment with an EGFR modulator, with Compound A or a pharmaceutically acceptable salt thereof. Possible EGFR modulators that upon treatment can result in resistance are, for instance, gefitinib, erlotinib, lapatinib, cetuximab, nimotuzumab, panitumumab, and trastuzumab.

Thus, the present invention provides the use of Compound A, or a tautomer thereof, or a pharmaceutically acceptable salt, or a hydrate or solvate thereof for the manufacture of a pharmaceutical preparation for the treatment of an EGFR dependent disease which is:
- non small cell lung carcinoma
- head and neck cancer
- colorectal carcinoma
- breast cancer
- brain malignancies including glioblastoma
- prostate cancer
- bladder cancer
- renal cell carcinoma
- pancreas cancer
- cervical cancer
- esophageal cancer
- gastric cancer
- ovarian cancer
or any combination thereof,
wherein said disease is resistant to the treatment with an EGFR modulator selected from the group consisting of gefitinib, erlotinib, lapatinib, cetuximab, nimotuzumab, panitumumab and trastuzumab.

2-methyl-2-[4-(3-methyl-2-oxo-8-quinolin-3yl-2,3-dihydro-imidazo[4,5-c]quinolin-1-yl)-phenyl]propionitrile (Compound A), or a tautomer thereof, or a pharmaceutically acceptable salt, or a hydrate or solvate thereof, may also be used for the treatment of the EGFR dependent diseases listed above in combination with an EGFR modulator selected from the group consisting of gefitinib, erlotinib, lapatinib, NVP-AEE778, ARRY334543, BIRW2992,BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010; CNF2024, tanespimycinm alvespimycin, IPI504, SNX5422 and NVP-AUY922`

The present invention also relates to a combination treatment of EGFR dependent diseases with 2-methyl-2-[4-(3-methyl-2-oxo-8-quinolin-3-yl-2,3-dihydro-imidazo[4,5-c]quinolin-1-yl)-phenyl]-propionitrile (Compound A) and an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922, wherein the active ingredients are present in each case in free form or in the form of a pharmaceutically acceptable salt, and optionally at least one pharmaceutically acceptable carrier, for simultaneous, separate or sequential use for the treatment of non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and/or ovarian cancer

In particular, the present invention relates to a combination of 2-methyl-2-[4-(3-methyl-2-oxo-8-quinolin-3-yl-2,3-dihydro-imidazo[4,5-c]quinolin-1-yl)-phenyl]-propionitrile and an EGFR modulator selected from the group consisting of gefitinib, erlotinib, lapatinib, cetuximab, nimotuzumab, panitumumab, and trastuzumab, wherein the active ingredients are present in each case in free form or in the form of a pharmaceutically acceptable salt, and optionally at least one pharmaceutically acceptable carrier; for simultaneous, separate or sequential use for the treatment of non small cell lung carcinoma, head and neck cancer, colorectal carcinoma, breast cancer, brain malignancies including glioblastoma, prostate cancer, bladder cancer, renal cell carcinoma, pancreas cancer, cervical cancer; esophageal cancer, gastric cancer and ovarian cancer.

The present disclosure also describes a method of treating an EGFR dependent disease or a malignancy, preferably a malignancy, that has acquired resistance to EGFR kinase modulators during treatment with said EGFR modulator, comprising administering a therapeutically effective amount of a specific imidazoquinoline derivative of 2-methyl-2-[4-(3-methyl-2-oxo-8-quinolin-3-yl-2,3-dihydro-imidazo[4,5-c]quinolin-1-yl)-phenyl]-propionitrile (Compound A) or a pharmaceutically acceptable salt thereof, alone or in combination with an EGFR modulator, to a warm-blooded animal in need thereof.

The diseases to be treated by this method are non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and ovarian cancer.

In another embodiment the present invention relates to a pharmaceutical preparation for the treatment of an EGFR dependent disease or a disease that has acquired resistance during treatment with an EGFR modulator comprising 2-methyl-2-[4-(3-methyl-2-oxo-8-quinolin-3-yl-2,3-dihydro-imidazo[4_{;}5-c]quinolin-1-yl)-phenyl]-propionitrile (Compound A), or a pharmaceutically acceptable salt thereof and at least one pharmaceutically acceptable carrier, alone or in combination with an EGFR modulator; wherein
the diseases to be treated by this pharmaceutical preparation are non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and ovarian cancer.

The present disclosure describes to the use of a 2-methyl-2-[4-(3-methyl-2-oxo-8-quinolin-3-yl-2,3-dihydro-imidazo[4,5-c]quinolin-1-yl)-phenyl]-propionitrile (Compound A), or a pharmaceutically acceptable salt thereof for the treatment of an EGFR dependent disease or disease that has acquired resistance during treatment with an EGFR modulator.

The diseases to be treated by these compounds, alone or in combination with an EGFR modulator, are non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and ovarian cancer.

The compound 2-methyl-2-[4-(3-methyl-2-oxo-8-quinolin-3-yl-2,3-dihydro-imidazo[4,5-c]quinolin-1-yl)-phenyl]-propionitrile (Compound A) or a tautomer thereof, or a pharmaceutically acceptable salt, or a hydrate or solvate thereof, may also be used to advantage in combination with known therapeutic processes, for example, the administration of hormones or, especially, radiation. A compound of formula (I) may in particular be used as a radiosensitizer, especially for the treatment of tumors which exhibit poor sensitivity to radiotherapy.

Treatment in accordance with the invention may be symptomatic or prophylactic.

By "combination", there is meant either a fixed combination in one dosage unit form, or a kit of parts for the combined administration where
the compound 2-methyl-2-[4-(3-methyl-2-oxo-8-quinolin-3-yl-2,3-dihydro-imidazo[4,5-c]quinolin-1-yl)-phenyl]-propionitrile (Compound A) or a tautomer thereof, or a pharmaceutically acceptable salt, or a hydrate or solvate thereof,
and a combination partner may be administered independently at the same time or separately within time intervals that especially allow that the combination partners show a cooperative, e.g. synergistic effect.

The compound 2-methyl-2-[4-(3-methyl-2-oxo-8-quinolin-3-yl-2,3-dihydro-imidazo[4,5-c]quinolin-1-yl)-phenyl]-propionitrile (Compound A) or a tautomer thereof, or a pharmaceutically acceptable salt, or a hydrate or solvate thereof,
can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds.

The dosage of the active ingredient depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

The compound_2-methyl-2-[4-(3-methyl-2-oxo-8-quinolin-3-yl-2,3ydihydro-imidazo[4,5-c]quinolin-1-yl)-phenyl]-propionitrile (Compound A) or a tautomer thereof, or a pharmaceutically acceptable salt, or a hydrate or solvate thereof, may be administered by any conventional route, in particular parenterally, for example in the form of injectable solutions or suspensions, enterally, e.g. orally, for example in the form of tablets or capsules, topically, e.g. in the form of lotions, gels, ointments or creams, or in a nasal or a suppository form. Topical administration is e.g. to the skin. A further form of topical administration is to the eye. Pharmaceutical compositions comprising the compound 2-methyl-2-[4-(3-methyl-2-oxo-8-quinolin-3-yl-2,3-dihydro-imidazo[4,5-c]quinolin-1-yl)-phenyl]-propionitrile (Compound A) or a tautomer thereof, or a pharmaceutically acceptable salt, or a hydrate or solvate thereof, in association with at least one pharmaceutical acceptable carrier or diluent may be manufactured in conventional manner by mixing with a pharmaceutically acceptable carrier or diluent.

The pharmaceutical compositions are comprising an amount effective in the treatment of one of the above-mentioned disorders, of the compound 2-methyl-2-[4-(3-methyl-2-oxo-8-quinolin-3-yl-2,3-dihydro-imidazo[4,5-c]quinolin-1-yl)-phenyl]propionitrite (Compound A) or an N-oxide or a tautomer thereof together with pharmaceutically acceptable carriers that are suitable for topical, enteral, for example oral or rectal, or parenteral administration and that may be inorganic or organic, solid or liquid. There are pharmaceutical compositions used for oral administration especially tablets or gelatin capsules that comprise the active ingredient together with diluents, for example lactose, dextrose, manitol, and/or glycerol, and/or lubricants and/or polyethylene glycol. Tablets may also comprise binders, for example magnesium aluminum silicate, starches, such as corn, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate; and/or effervescent mixtures, or adsorbents, dyes, flavorings and sweeteners. It is also possible to use the pharmacologically active compounds of the present invention in the form of parenterally administrable compositions or in the form of infusion solutions. The pharmaceutical compositions may be sterilized and/or may comprise excipients, for example preservatives, stabilizers, wetting compounds and/or emulsifiers, solubilisers, salts for regulating the osmotic pressure and/or buffers. The present pharmaceutical compositions, which may, if desired, comprise other pharmacologically active substances are prepared in a manner known per se, for example by means of conventional mixing, granulating, confectioning, dissolving or lyophilising lyophilizing processes, and comprise approximately from 1% to 99%, especially from approximately 1% to approximately 20%, active ingredient(s).

## Claims

1. Use of the compound 2-methyl-2-[4-(3-methyl-2-oxo-8-quinolin-3-yl-2,3-dihydro-imidazo[4,5-c]quinolin-1-yi)-phenyl]-propionitrile (Compound A) or a tautomer thereof, or a pharmaceutically acceptable salt, or a hydrate or solvate thereof, for the manufacture of a pharmaceutical preparation for the treatment of an EGFR dependent disease, wherein the disease to be treated is
• non small cell lung carcinoma
• head and neck cancer
• colorectal carcinoma
• breast cancer
• brain malignancies including glioblastoma
• prostate cancer
• bladder cancer
• renal cell carcinoma
• pancreas cancer
• cervical cancer
• esophageal cancer
• gastric cancer
• ovarian cancer
or any combination thereof.

2. The use according to claim 1, wherein said disease is resistant to the treatment with an EGFR modulator selected from the group consisting of gefitinib, erlotinib, lapatinib, cetuximab, nimotuzumab, panitumumab and trastuzumab.

3. The use according to claim 2, wherein resistance to the treatment with an EGFR modulator has been acquired during treatment with said EGFR modulator.

4. The use according to claim 3, wherein the resistance is due to a mutation or mutations in the protein.

5. The use according to claim 1, together with an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8; pertuzumab, trastuzumab, zemab®, the Her2 vaccine PX 1041, and the HSP90 inhibitors CNF1 01 0, CNF2024, tanespimycinm alvespimycin, IP1504, SNX5422 and NVP-AUY922.

6. Combination of the compound 2-methyl-2-[4-(3-methyl-2-oxo-8-quinolin-3-yl-2,3-dihydro-imidazo[4,5-c]quinolin-1-yl)-phenyl]-propionitrile (Compound A) and an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab; MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922, wherein the active ingredients are present in each case in free form or in the form of a pharmaceutically acceptable salt, and optionally at least one pharmaceutically acceptable carrier, for simultaneous, separate or sequential use for the treatment of non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and/or ovarian cancer.

7. A pharmaceutical preparation for use in the treatment of an EGFR dependent disease or a disease that has acquired resistance during treatment with an EGFR modulator, wherein the disease to be treated is non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer, ovarian cancer or any combination thereof, comprising a compound according to claim 1 or a pharmaceutically acceptable salt thereof and at least one pharmaceutically acceptable carrier.

8. The pharmaceutical preparation for the use according to claim 7, further comprising an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab; zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922.

9. A compound, according to claim 1 or a pharmaceutically acceptable salt thereof, for use in the treatment of an EGFR dependent disease or disease that has acquired resistance during treatment with an EGFR modulator, wherein the disease to be treated is non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer, ovarian cancer or any combination thereof.

## Patentansprüche

1. Verwendung der Verbindung 2-Methyl-2-[4-(3-methyl-2-oxo-8-chinolin-3-yl-2,3-dihydro-imidazo[4,5-c]chinolin-1-yl)-phenyl]propionitril (Verbindung A) oder eines Tautomers hiervon oder eines pharmazeutisch akzeptablen Salzes oder eines Hydrats oder eines Solvats hiervon zur Herstellung einer pharmazeutischen Zubereitung zur Behandlung einer EGFR-abhängigen Erkrankung, wobei die zu behandelnde Erkrankung
- nicht kleinzelliges Lungenkarzinom
- Kopf- und Halskrebs
- kolorektales Karzinom
- Brustkrebs
- Hirnmalignitäten einschließlich Glioblastom
- Prostatakrebs
- Blasenkrebs
- Nierenzellkarzinom
- Pankreaskrebs
- Gebärmutterhalskrebs
- Speiseröhrenkrebs
- Magenkrebs
- Eierstockkrebs
oder eine beliebige Kombination hiervon ist.

2. Verwendung nach Anspruch 1, wobei die Erkrankung gegenüber einer Behandlung mit einem EGFR-Modulator resistent ist, der aus der Gruppe ausgewählt ist, die aus Gefitinib, Erlotinib, Lapatinib, Cetuximab, Nimotuzumab, Panitumumab und Trastuzumab besteht.

3. Verwendung nach Anspruch 2, wobei die Resistenz gegenüber der Behandlung mit einem EGFR-Modulator während der Behandlung mit dem EGFR-Modulator erworben wurde.

4. Verwendung nach Anspruch 3, wobei die Resistenz auf eine Mutation oder Mutationen in dem Protein zurückzuführen ist.

5. Verwendung nach Anspruch 1, zusammen mit einem EGFR-Modulator, der aus der Gruppe ausgewählt ist, die aus Gefitinib, Erlotinib, Lapatinib, NVP-AEE778, ARRY334543, BIRW2992, BMS690514, Pelitinib, Vandetanib, AV412, dem monoklonalen anti-EGFR-Antikörper 806, dem monoklonalen anti-EGFR-Antikörper Y90/Re-188, Cetuximab, Panitumumab, Matuzumab, Nimotuzumab, Zalutumumab, Pertuzumab, MDX-214, CDX110, IMC11F8, Pertuzumab, Trastuzumab, Zemab^{®}, dem Her2-Vakzin PX1041 und den HSP90-Inhibitoren CNF1010, CNF2024, Tanespimycin, Alvespimycin, IPI504, SNX5422 und NVP-AUY922 besteht.

6. Kombination der Verbindung 2-Methyl-2-[4-(3-methyl-2-oxo-8-chinolin-3-yl-2,3-dihydro-imidazo[4,5-c]chinolin-1-yl)-phenyl]propionitril (Verbindung A) und eines EGFR-Modulators, der aus der Gruppe ausgwählt ist, die aus Gefitinib, Erlotinib, Lapatinib, NVP-AEE778, ARRY334543, BIRW2992, BMS690514, Pelitinib, Vandetanib, AV412, dem monoklonalen anti-EGFR-Antikörper 806, dem monoklonalen anti-EGFR-Antikörper Y90/Re-188, Cetuximab, Panitumumab, Matuzumab, Nimotuzumab, Zalutumumab, Pertuzumab, MDX-214, CDX110, IMC11F8, Pertuzumab, Trastuzumab, Zemab^{®}, dem Her2-Vakzin PX1041 und den HSP90-Inhibitoren CNF1010, CNF2024, Tanespimycin, Alvespimycin, IPI504, SNX5422 und NVP-AUY922 besteht, wobei die wirksamen Bestandteile in jedem Fall in freier Form oder in Form eines pharmazeutisch akzeptablen Salzes vorhanden sind, und optional mindestens eines pharmazeutisch akzeptablen Trägers zur gleichzeitigen, getrennten oder aufeinanderfolgenden Verwendung bei der Behandlung von nicht kleinzelligem Lungenkarzinom, Kopf- und Halskrebs, kolorektalem Karzinom, Brustkrebs, Hirnmalignitäten einschließlich Glioblastom, Prostatakrebs, Blasenkrebs, Nierenzellkarzinom, Pankreaskrebs, Gebärmutterhalskrebs, Speiseröhrenkrebs, Magenkrebs und/oder Eierstockkrebs.

7. Pharmazeutische Zubereitung zur Verwendung bei der Behandlung einer EGFR-abhängigen Erkrankung oder einer Erkrankung, die eine Resistenz während der Behandlung mit einem EGFR-Modulator erworben hat, wobei die zu behandelnde Erkrankung nicht kleinzelliges Lungenkarzinom, Kopf- und Halskrebs, kolorektales Karzinom, Brustkrebs, Hirnmalignitäten einschließlich Glioblastom, Prostatakrebs, Blasenkrebs, Nierenzellkarzinom, Pankreaskrebs, Gebärmutterhalskrebs, Speiseröhrenkrebs, Magenkrebs, Eierstockkrebs oder eine beliebige Kombination hiervon ist, wobei die pharmazeutische Zubereitung eine Verbindung nach Anspruch 1 oder ein pharmazeutisch akzeptables Salz hiervon und mindestens einen pharmazeutisch akzeptablen Träger umfasst.

8. Pharmazeutische Zubereitung zur Verwendung nach Anspruch 7, die des Weiteren einen EGFR-Modulator umfasst, der aus der Gruppe ausgewählt ist, die aus Gefitinib, Erlotinib, Lapatinib, NVP-AEE778, ARRY334543, BIRW2992, BMS690514, Pelitinib, Vandetanib, AV412, dem monoklonalen anti-EGFR-Antikörper 806, dem monoklonalen anti-EGFR-Antikörper Y90/Re-188, Cetuximab, Panitumumab, Matuzumab, Nimotuzumab, Zalutumumab, Pertuzumab, MDX-214, CDX110, IMC11F8, Pertuzumab, Trastuzumab, Zemab^{®}, dem Her2-Vakzin PX1041 und den HSP90-Inhibitoren CNF1010, CNF2024, Tanespimycin, Alvespimycin, IPI504, SNX5422 und NVP-AUY922 besteht.

9. Verbindung nach Anspruch 1 oder ein pharmazeutisch akzeptables Salz hiervon zur Verwendung bei der Behandlung einer EGFR-abhängigen Erkrankung oder einer Erkrankung, die eine Resistenz während der Behandlung mit einem EGFR-Modulator erworben hat, wobei die zu behandelnde Erkrankung nicht kleinzelliges Lungenkarzinom, Kopf- und Halskrebs, kolorektales Karzinom, Brustkrebs, Hirnmalignitäten einschließlich Glioblastom, Prostatakrebs, Blasenkrebs, Nierenzellkarzinom, Pankreaskrebs, Gebärmutterhalskrebs, Speiseröhrenkrebs, Magenkrebs, Eierstockkrebs oder eine beliebige Kombination hiervon ist.

## Revendications

1. Utilisation du composé 2-méthyl-2-[4-(3-méthyl-2-oxo-8-quinoléin-3-yl-2,3-dihydro-imidazo[4,5-c]quinoléin-1-yl)phényl]propionitrile (Composé A) ou d'un tautomère de celui-ci, ou de l'un de leurs sels, hydrates ou solvates acceptables sur le plan pharmaceutique, pour la fabrication d'une préparation pharmaceutique destinée au traitement d'une maladie dépendante d'EGFR, ladite maladie à traiter étant
• le cancer du poumon non à petites cellules
• le cancer de la tête et du cou
• le cancer colorectal
• le cancer du sein
• les tumeurs malignes du cerveau, y compris le glioblastome
• le cancer de la prostate
• le cancer de la vessie
• le cancer des cellules rénales
• le cancer du pancréas
• le cancer du col de l'utérus
• le cancer de l'oesophage
• le cancer de l'estomac
• le cancer des ovaires
ou une combinaison quelconque de ces maladies.

2. Utilisation selon la revendication 1, où ladite maladie est résistante au traitement avec un modulateur d'EGFR choisi dans le groupe constitué par le géfitinib, l'erlotinib, le lapatinib, le cétuximab, le nimotuzumab, le panitumumab et le trastuzumab.

3. Utilisation selon la revendication 2, où la résistance au traitement avec un modulateur d'EGFR a été acquise lors du traitement avec ledit modulateur d'EGFR.

4. Utilisation selon la revendication 3, où la résistance est due à une ou plusieurs mutations dans la protéine.

5. Utilisation selon la revendication 1, conjointement avec un modulateur d'EGFR choisi dans le groupe constitué par le géfitinib, l'erlotinib, le lapatinib, NVP-AEE778, ARRY334543, BIRW2992, BMS690514, le pélitinib, le vandétanib, AV412, l'anticorps monoclonal anti-EGFR 806, l'anticorps monoclonal anti-EGFR-Y90/Re-188, le cétuximab, le panitumumab, le matuzumab, le nimotuzumab, le zalutumumab, le pertuzumab, MDX-214, CDX110, IMC11F8, le pertuzumab, le trastuzumab, le zémab®, le vaccin Her2 PX 1041, et les inhibiteurs de HSP90 CNF1010, CNF2024, tanespimycine alvespimycine, IPI504, SNX5422 et NVP-AUY922.

6. Combinaison du composé 2-méthyl-2-[4-(3-méthyl-2-oxo-8-quinoléin-3-yl-2,3-dihydro-imidazo[4,5-c]quinoléin-1-yl)phényl]-propionitrile (Composé A) et d'un modulateur d'EGFR choisi dans le groupe constitué par le géfitinib, l'erlotinib, le lapatinib, NVP-AEE778, ARRY334543, BIRW2992, BMS690514, le pélitinib, le vandétanib, AV412, l'anticorps monoclonal anti-EGFR 806, l'anticorps monoclonal anti-EGFR-Y90/Re-188, le cétuximab, le panitumumab, le matuzumab, le nimotuzumab, le zalutumumab, le pertuzumab, MDX-214, CDX110, IMC11F8, le pertuzumab, le trastuzumab, le zémab®, le vaccin Her2 PX 1041, et les inhibiteurs de HSP90 CNF1010, CNF2024, tanespimycine alvespimycine, IPI504, SNX5422 et NVP-AUY922, dans laquelle les principes actifs sont présents dans chaque cas sous forme libre ou sous la forme d'un sel acceptable sur le plan pharmaceutique, avec facultativement, au moins un véhicule acceptable sur le plan pharmaceutique, en vue d'une utilisation simultanée, séparée ou séquentielle pour le traitement du cancer du poumon non à petites cellules, du cancer de la tête et du cou, du cancer colorectal, du cancer du sein, des tumeurs malignes du cerveau, y compris le glioblastome, du cancer de la prostate, du cancer de la vessie, du cancer des cellules rénales, du cancer du pancréas, du cancer du col de l'utérus, du cancer de l'oesophage, du cancer de l'estomac et/ou du cancer des ovaires.

7. Préparation pharmaceutique à utiliser dans le traitement d'une maladie dépendante d'EGFR ou d'une maladie qui a acquis une résistance lors du traitement avec un modulateur d'EGFR, ladite maladie à traiter étant le cancer du poumon non à petites cellules, le cancer de la tête et du cou, le cancer colorectal, le cancer du sein, les tumeurs malignes du cerveau, y compris le glioblastome, le cancer de la prostate, le cancer de la vessie, le cancer des cellules rénales, le cancer du pancréas, le cancer du col de l'utérus, le cancer de l'oesophage, le cancer de l'estomac, le cancer des ovaires ou une combinaison quelconque de ces maladies, comprenant un composé selon la revendication 1 ou l'un de ses sels acceptables sur le plan pharmaceutique, et au moins un véhicule acceptable sur le plan pharmaceutique.

8. Préparation pharmaceutique à utiliser selon la revendication 7, comprenant en outre un modulateur d'EGFR choisi dans le groupe constitué par le géfitinib, l'erlotinib, le lapatinib, NVP-AEE778, ARRY334543, BIRW2992, BMS690514, le pélitinib, le vandétanib, AV412, l'anticorps monoclonal anti-EGFR 806, l'anticorps monoclonal anti-EGFR-Y90/Re-188, le cétuximab, le panitumumab, le matuzumab, le nimotuzumab, le zalutumumab, le pertuzumab, MDX-214, CDX110, IMC11F8, le pertuzumab, le trastuzumab, le zémab®, le vaccin Her2 PX 1041, et les inhibiteurs de HSP90 CNF1010, CNF2024, tanespimycine alvespimycine, IPI504, SNX5422 et NVP-AUY922.

9. Composé selon la revendication 1 ou l'un de ses sels acceptables sur le plan pharmaceutique, à utiliser dans le traitement d'une maladie dépendante d'EGFR ou d'une maladie qui a acquis une résistance lors du traitement avec un modulateur d'EGFR, où la maladie à traiter est le cancer du poumon non à petites cellules, le cancer de la tête et du cou, le cancer colorectal, le cancer du sein, les tumeurs malignes du cerveau, y compris le glioblastome, le cancer de la prostate, le cancer de la vessie, le cancer des cellules rénales, le cancer du pancréas, le cancer du col de l'utérus, le cancer de l'oesophage, le cancer de l'estomac, le cancer des ovaires ou une combinaison quelconque de ces maladies.
